# EUROPEAN PATENT SPECIFICATION

(11) **EP 1 504 104 B1**
(45) Date of publication and mention of the grant of the patent: **12.08.2009**
(21) Application number: 03722597.6
(22) Date of filing: 29.04.2003
(51) Int. Cl.: C12N 15/82, C07K 14/325, A01H 5/00

(54) **INSECT RESISTANT PLANTS AND METHODS FOR MAKING THE SAME**
INSEKTRESISTENTE PFLANZEN UND VERFAHREN ZU DEREN HERSTELLUNG
PLANTES RESISTANT AUX INSECTES ET PROCEDES DE FABRICATION CORRESPONDANTS

(30) Priority: 03.05.2002 US 137682
(43) Date of publication of application: 09.02.2005
(73) Proprietor: Bayer BioScience N.V., 9052 Gent (BE)
(72) Inventor: JANSENS, Stefan, B-9000 Gent (BE); VAN HOUDT, Sara, B-9620 Zottegem (BE); REYNAERTS, Arlette, B-9031 Drongen (BE)
(86) International application number: PCT/EP2003/004700
(87) International publication number: WO 2003/093484

(56) References cited:
- EP-A- 0 451 878
- US-A- 5 500 365
- US-A- 5 595 733
- US-A- 6 114 608
- KOZIEL M G ET AL: "FIELD PERFORMANCE OF ELITE TRANSGENIC MAIZE PLANTS EXPRESSING AN INSECTICIDAL PROTEIN DERIVED FROM BACILLUS THURINGIENSIS" BIO/TECHNOLOGY, NATURE PUBLISHING CO. NEW YORK, US, vol. 11, 1 February 1993 (1993-02-01), pages 194-200, XP002025246 ISSN: 0733-222X -& WO 93 07278 A (CIBA GEIGY AG) 15 April 1993 (1993-04-15)
- HOEFTE H ET AL: "STRUCTURAL AND FUNCTIONAL ANALYSIS OF A CLONED DELTA ENDOTOXIN OF BACILLUS THURINGIENSIS BERLINER 1715" EUROPEAN JOURNAL OF BIOCHEMISTRY, BERLIN, DE, vol. 161, no. 2, 1 December 1986 (1986-12-01), pages 273-280, XP000572684 ISSN: 0014-2956 cited in the application
- HOFTE H ET AL: "INSECTICIDAL CRYSTAL PROTEINS OF BACILLUS THURINGIENSIS" MICROBIOLOGICAL REVIEWS, AMERICAN SOCIETY FOR MICROBIOLOGY, WASHINGTON, DC, US, vol. 53, 1 June 1989 (1989-06-01), pages 242-255, XP000374163 ISSN: 0146-0749

## Description

### FIELD OF THE INVENTION

The present invention relates to a DNA sequence encoding a modified CrylAb protein that has insecticidal activity. The invention further relates to a method for producing insect resistant plants by introducing into the genome of the plants a foreign DNA comprising such a modified cry1Ab coding sequence. The invention further relates to plants or parts thereof comprising in their genome the modified cry1Ab coding sequence of the present invention.

### BACKGROUND ART

The Gram-positive soil bacterium *Bacillus thuringiensis* is well known for its production of proteins or delta-endotoxins, that are toxic to a variety of lepidopteran, coleopteran, and dipteran larvae. Different strains of *B. thuringiensis* have been shown to produce different insecticidal crystal proteins, which are specifically toxic to certain species of insects (reviewed by Höfte and Whiteley, 1989; Schnepf et al., 1998).

The specific toxicity of insecticidal toxins produced by *B. thuringiensis* for target insects and their non-toxicity to plants and other organisms has made compositions comprising different Bt strains the product of choice for the biological control of agricultural insect pests. Various of the genes encoding the crystal proteins have been cloned and their DNA sequences determined (Höfte and Whiteley, 1989; Crickmore et al., 1995). This has led to the engineering of modified delta-endotoxin encoding genes and the development of plants expressing these delta-endotoxin genes to make them insect resistant.

The family of *cry1* genes encode the Cryl crystal proteins, which are primarily active against lepidopteran pests. The pro-toxin form of Cryl delta-endotoxins consists of a C-terminal protoxin part which is not toxic and is thought to be important for crystal formation (Arvidson et al., 1989). The amino-half of the protoxin comprises the active toxin segment of the Cryl protein. Different domains have further been identified in the active toxin which are implied in different aspects of the toxicity effect (Grochulski et al., 1995). However, these functions seem to be dependent on the delta endotoxin examined.

Significant effort has gone into modifying the *cry1* genes so as to improve expression levels in plants while at least retaining their toxicity to the target insects. Modification of the crylAb and crylAc genes to remove putative plant polyadenylation signals and instability motifs (without altering the encoded amino acid sequences) resulted in increased resistance of the plants transformed with these sequences (van der Salm et al., 1994). Modifications of the cry1Ab gene have been described in US 6,320,100, US 6,180,774 and US 6114608. US 5,500,365 describes how modification in the 240 region of the coding region of a, *cry1Ab* gene so as to remove putative plant signals is of significant importance to increase expression levels and thereby toxicity of the Cry toxin in plants.

The present invention relates to a novel modified CrylAb protein and DNA sequences encoding this protein which can be used to engineer insect resistance in plants. More particularly, it was found that this modified sequence, despite having a native 240 region, ensures sufficiently high expression in plant cells to confer insect resistance to the plant or plant tissue in which it is expressed.

### SUMMARY OF THE INVENTION

The present invention relates to a modified crylAb coding sequence, which encodes the
wherein modified CrylAb protein of SEQ ID NO: 1, which is an insecticidal protein wherein. the DNA sequence encoding the modified CrylAb sequence comprises the sequence of SEQ ID NO:2.

The invention further relates to chimeric genes comprising the modified Cry1Ab DNA sequence of the present invention under the control of a plant-expressible promoter.

The invention further relates to recombinant vectors comprising the chimeric genes of the invention and to the production of transgenic plants using these recombinant vectors.

The invention further relates to plants and cells, seeds or tissues thereof, comprising in their genome a foreign DNA comprising the modified crylAb DNA sequence of the present invention under the control of a plant-expressible promoter. so as to protect said plants from lepidopteran insects, The invention also relates to a method for engineering insect resistance in plants, so as to protect said plants from lepidopteran insects by introducing by transforming , into the genome of the plant, a foreign DNA comprising the modified cry1Ab coding sequence of the present invention under the control of a plant-expressible promoter.

According to a particular embodiment of the present invention the modified cry1Ab coding sequence is particularly suited for engineering insect resistance in agricultural crops such as corn and cotton. Most particularly, expression of the modified Cry1Ab protein confers resistance to lepidopteran pests of these plants. More particularly, these pests include, but are not limited to, major lepidopteran pests of corn, cotton and rice, such as *Ostrinia nubilalis* (European corn borer or ECB), *Sesamia nonagrioides* (Mediterranean Stalk borer), *Sesamia inferens* (Pink stemborer), *Helicoverpa zea* (corn earworm, cotton bollworm), *Helicoverpa armigera* (American bollworm), *Heliothis virescens* (Tobacco budworm), *Scirpophaga incertulas* (Yellow stemborer), and *Cnaphalocrocis medinalis* (Rice leaf folder). Also provided herein is the use of the above chimeric gene to obtain insect-resistant plants.

### DETAILED DESCRIPTION

The term "gene" as used herein refers to any DNA sequence comprising several operable linked DNA fragments such as a promoter region, a 5' untranslated region (the 5 'UTR), a coding region, and an untranslated 3' region (3'UTR) comprising a polyadenylation site. A gene may include additional DNA fragments such as, for example, introns. While a promoter and a coding region are required in a gene used for plant transformation in the current invention, the 3' UTR comprising a polyadenylation site need not be present in the transferred gene itself, but can be recovered in the upstream plant DNA sequence after insertion of a gene not containing a 3' UTR comprising a polyadenylation site.

The term "chimeric" when referring to a gene or DNA sequence is used to refer to the fact that the gene or DNA sequence comprises at least two functionally relevant DNA fragments (such as promoter, 5'UTR, coding region, 3'UTR, intron) that are not naturally associated with each other and/or originate, for example, from different sources. "Foreign" referring to a gene or DNA sequence with respect to a plant species is used to indicate that the gene or DNA sequence is not naturally found in that plant, or is not naturally found in that genetic locus in that plant. The term "foreign DNA" will be used herein to refer to a DNA sequence as it has incorporated into the genome of a plant as a result of transformation.

A genome of a plant, plant tissue or plant cell, as used herein, refers to any genetic material in the plant, plant tissue or plant cell, and includes both the nuclear and the plastid and mitochondrial genome.

A "fragment" or "truncation" of a DNA molecule or protein sequence as used herein refers to a portion of the original DNA or protein sequence (nucleic acid or amino acid) referred to or a synthetic version thereof (such as a sequence which is adapted for optimal expression in plants), which can vary in length but which is sufficient to ensure the (encoded) protein is an insect toxin. A "variant" of a sequence is used herein to indicate a DNA molecule or protein of which the sequence (nucleic or amino acid) is essentially identical to the sequence to which the term refers. Sequences which are "essentially identical" means that when two sequences are aligned, the percent sequence identity, i.e. the number of positions with identical nucleotides or amino acids divided by the number of nucleotides or amino acids in the shorter of the sequences, is higher than 70%, preferably higher than 85%, more preferably higher than 90%, e specially preferably is higher than 95%, most preferably is between 96 and 100 %. The alignment of two nucleotide sequences is performed by the algorithm as described by Wilbur and Lipmann (1983) using a window size of 20 nucleotides, a word length of 4 nucleotides, and a gap penalty of 4.

A 'plant-expressible promoter' as used herein refers to a promoter which ensures expression of a coding sequence to which it is linked in a plant cell. Examples of such promoters are well known in the art. A plant-expressible promoter can be a constitutive promoter. Examples of promoters directing constitutive expression in plants are known in the art and include the 35S promoter from Cauliflower Mosaic virus, the nopaline synthase (NOS) promoter, the ubi promoter (Christensen et al. 1992), the promoter of the GOS2 gene from rice (de Pater et al., 1992). Alternatively, a plant-expressible promoter can be a tissue-specific promoter, i.e., a promoter directing a higher level of expression of a coding sequence (as can be measured by conventional RNA assays) in some tissues of the plant, e.g. in green tissues (such as the promoter of the PEP carboxylase) than in other t issues of t he p lant. Alternatively, a p lant-expressible promoter can be a wound-inducible promoter. A 'wound-inducible' promoter or a promoter directing an expression pattern which is wound-inducible as used herein means that upon wounding of the plant, either mechanically or by insect feeding, expression of the coding sequence under control of the promoter is significantly increased. Examples of wound-inducible promoters include the proteinase inhibitor gene of potato and tomato (pin1 and pin2)(Johnson et al., 1989) and the promoter of the maize proteinase inhibitor (MPI) gene (Cordero et al. 1994).

The "TR2' promoter" as used herein relates to any promoter comprising the TR2' (or mannopine synthase, abbreviated as *mas)* functional part of the TR1'-TR2' dual promoter element from Agrobacterium (Velten et al. 1984; Langridge et al. 1989). Thus, this can comprise the TR2' element either alone or in combination with all or part of the divergent TR1' element (Guevara-Garcia et al., 1998) or other (regulatory) elements, including but not limited to enhancer regions, introns and the like, as long as the wound-induction characteristics in monocots, particularly corn, in accordance with the present invention are substantially retained. In a preferred embodiment of this invention, transcription is directed from the TR2' promoter region (and the coding sequence is hence downstream of the TR2' promoter sequence), even if the TR1'-TR2' dual promoter (or any part thereof retaining the Tor2' promoter element) is used.

'Insecticidal' is used herein to mean toxic to insects which are crop pests. More particularly, in the context of the present invention target insects are the pests such as, but not limited to, major lepidopteran pests, such as *Ostrinia nubilalis* (European corn borer or ECB), *Sesamia nonagrioides* (Mediterranean Stalk borer), *Helicoverpa zea* (corn earworm, cotton bollworm), *Helicoverpa armigera* (American bollworm) and *Heliothis viriscens* (Tobacco budworm).

In a preferred embodiment of the present invention, the DNA encoding an insecticidal crystal protein (ICP) is a modified cry1Ab DNA sequence encoding an ICP which correspons to the sequence of SEQ ID NO: 1.,
wherein the modified crylAb coding sequence comprises the sequence of SEQ ID NO: 2. The DNA sequence used for transformation of plants, particularly corn, cotton or rice, in accordance with the current invention, can also comprise other elements besides a coding region encoding the modified CrylAb protein of the invention, such as a coding region encoding a transit peptide, a coding region encoding a selectable marker protein or a protein conferring resistance to a herbicide.

In a preferred embodiment of the invention the modified CrylAb protein is toxic to major lepidopteran pests of crops such as corn, cotton and rice. The plants of the present invention, the above comprising a foreign DNA in their genome comprising the above DNA encoding a modified CrylAb protein are protected against these pests, by expressing a controlling amount of this protein. By controlling is meant a toxic (lethal) or combative (sublethal) amount. At the same time, the plants should be morphologically normal and may be cultivated in a usual manner for consumption and/or production of products. Furthermore, said plants should substantially obviate the need for chemical or biological insecticides (to insects targetted by the modified CrylAb protein).

The expression level of an ICP in plant material can be determined in a number of ways described in the art, such as by quantification of the mRNA encoding the insecticidal protein produced in the tissue using specific primers (such as described by Cornelissen & Vandewiele, 1989) or direct specific detection of the amount of insecticidal protein produced, e.g., by immunological detection methods. More particularly, according to the present invention the expression level of a modified Cry1Ab protein is represented as the percentage of soluble insecticidal protein as determined by immunospecific ELISA as described herein related to the total amount of soluble protein (as determined, e.g., by Bradford analysis (Bradford, 1976)). A preferred ELISA in the current invention is a sandwich ELISA (Clark et al., 1986).

Different assays can be used to measure the insecticidal effect or efficacy of ICP expression in the plant. According to the present invention, the target insects are the major lepidopteran pests of agricultural crops such as corn, cotton and rice, more particularly the European Corn Borer (ECB) and Sesamia nonagrioides (SMG) in corn, the cotton bollworm (CBW) and tobacco budworm (TBW) in cotton, and the yellow stem borer, the pink stem borer and the rice leaf folder in rice. The toxicity of an ICP produced in a corn plant on ECB can be assayed *in vitro* by testing of protein extracted from the plant in feeding bioassays with ECB larvae or by scoring mortality of larvae distributed on leaf material of transformed plants in a petri dish (both assays as described by Jansens et al., 1997), or on plants isolated in individual cylinders. In the field first brood European corn borer (ECB1) infestation is evaluated based on leaf damage ratings (Guthrie, 1989) while evaluation of the total number of stalk tunnels per plant and stalk tunnel length are indicative of second brood (ECB2) stalk feeding damage.
Efficacy of the ICP produced in cotton plants transformed with a modified crylAb gene can similarly be measured using in vitro and/or in vivo assays. Toxicity of the transformed plant tissue to CBW larvae can be measured by feeding CBW larvae on squares, leaves or terminals and assaying weight of surviving larvae. In the field, plants are artificially infested with neonate CBW larvae and rating damage to leaves, terminals, squares, white bloom and bolls at regular intervals (as described herein). It will be understood that similar assays can be developed for any target or non-target insect in order to determine efficacy of the ICP produced in the plant against such insect.

The plants of the present invention optionally also comprise in their genome a gene encoding herbicide resistance. More particularly, the herbicide resistance gene is the bar or the pat gene, which confers glufosinate tolerance to the plant, i.e. the plants are tolerant to the herbicide LibertyTM. Tolerance to LibertyTM can be tested in different ways. For instance, tolerance can be tested by LibertyTM spray application. Spray treatments should be made between the plant stages V2 and V6 for best results (corn stages are as determined in 'How a Corn Plant Develops, Special Report No. 48, Iowa State University of Science and Technology, Cooperative Extension Service, Ames, Iowa, Reprinted June 1993; s ee also the website a t: http://www.extension.iastate.edu/pages/hancock/agriculture/corn/corn develop/CornGrowthSt ages.html:). Tolerant plants are characterized by the fact that spraying of the plants with at least 200 grams active ingredient/hectare (g.a.i./ha), preferably 400 g.a.i./ha, and possibly up to 1600 g.a.i./ha (4X the normal field rate), does not kill the plants. A broadcast application should be applied at a rate of 28-34 oz LibertyTM + 3 1b Ammonium Sulfate per acre. It is best to apply at a volume of 20 gallons of water per acre using a flat fan type nozzle while being careful not to direct s pray applications directly into the whor1 of the plants t o a void surfactant burn on the leaves. The herbicide effect should appear within 48 hours and be clearly visible within 5-7 days.

Examples of other herbicide resistance genes are the genes encoding resistance to phenmedipham (such as the pmph gene, US 5,347,047; US 5,543,306), the genes encoding resistance to glyphosate (such as the EPSPS genes, US 5,510,471), genes encoding bromoxynyl resistance (such as described in US 4,810,648), genes encoding resistance to sulfonylurea (such as described in EPA 0 360 750), genes encoding resistance to the herbicide dalapon (such as described in WO 99/27116), and genes encoding resistance to cyanamide (such as described in W O 98/48023 and WO 98/56238), and genes encoding resistance to glutamine synthetase inhibitors, such as PPT (such as described in EP-A-0 242 236, EP-A-0 242 246, EP-A-0 257 542).

Introduction of a foreign DNA into a plant cell can be obtained by conventional transformation methods described in the art. Such methods include but are not limited to *Agrobacterium* mediated transformation (US 6,074,877, Hiei et al., 1997), microprojectile bombardment (as described, for example by Chen et al., 1994; Casas et al., 1995; Christou, 1997, Finer et al., 1999, Vasil et al. 1999), direct DNA uptake into protoplasts (as described, for example by De Block et al. 1989; Poulsen, 1996, Datta et al., 1999), electroporation (D'Halluin et al., 1992, US 5,641,665; Bates, 1995) or silicon whisker mediated DNA introduction (Dunwell, 1999) or other methods as generally reviewed by Potrykus (1990), Sawahel et al. (1995), Komari et al. (1998), Bogorad (2000) and Newell (2000).

The following examples describe the development of a DNA sequence encoding a modified Cry1Ab protein, the construction of chimeric genes comprising this sequence for expression in plants, and insect resistant plants obtained therewith. Unless stated otherwise in the Examples, all recombinant DNA techniques are carried out according to standard protocols as described in Sambrook and Russell (2001) Molecular Cloning: A Laboratory Manual, Third Edition, Cold Spring Harbor Laboratory Press, NY, in Volumes 1 and 2 of Ausubel et al. (1994) Current Protocols in Molecular Biology, Current Protocols, USA and in Volumes I and II of Brown (1998) Molecular Biology LabFax, Second E dition, Academic Press (UK). Standard materials and methods for plant molecular work are described in Plant Molecular Biology Labfax (1993) by R.D.D. Croy, jointly published by BIOS Scientific Publications Ltd (UK) and Blackwell Scientific Publications, UK. Standard materials and methods for polymerase c hain reactions c an be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

Throughout the description and Examples, reference is made to the following sequences represented in the sequence listing:

| | |
|---|---|
| SEQ ID NO: 1: | Modified Cry1Ab protein |
| SEQ ID NO: 2: | DNA sequence encoding a modified CrylAb protein |

### EXAMPLES

### 1. Development of a modified Cry1Ab gene

The modified CrylAb DNA sequence used herein encodes part of the CrylAb5 protein described by Höfte et al. (1986) corresponding to amino acid 1 to 616, which has an insertion of an alanine codon (GCT) 3' of the ATG start codon (AlaAsp2...Asp616). The protein sequence of the modified Cry1Ab protein is provided in SEQ ID NO: 1. The sequence of the DNA encoding such a modified CrylAb protein is provided in SEQ ID NO: 2.

### 2. Cry1Ab gene in corn

### I. Development of CrylAb events in corn

For *Agrobacterium* transformation of corn, constructs were developed wherein the crylAb coding sequence was placed under the control of different promoters: 35S promoter (Odell et al. 1985), the ubi promoter (Christensen et al. 1992), the promoter of the GOS2 gene from rice (de Pater et al., 1992) with the cab22 leader from Petunia (Harpster et al. 1988), the 5' leader sequence of the GOS2 gene from rice, containing the second exon, the first intron and the first exon of the GOS transcript (de Pater et al., 1992), or a TR2' promoter region (Velten et al. 1984); all constructs included the 35S-bar gene. Agrobacterium-mediated transformation was done by co-cultivation of type I callus derived from immature embryo's with strain C58Cl (pTiEHA101)(pTTS35)(Agrobacterium C58ClRifR strain cured for pTiC58 harboring the non-oncogenic Ti plasmid pTiEHA101 (Hood et al., 1986) and the plasmids in the table below containing the genes of interest placed between the T-DNA borders). Protoplast transformation was done by PEG mediated transfection of protoplasts prepared from suspension cultures derived from Pa91xH99xHE89 Z15 embryos. The DNA used for transfection was a purified fragment of the plasmids in the table below containing the genes of interest between T-DNA borders.

| **Agrobacterium transformation** | Construct description | Abbreviation |
|---|---|---|
| PTSVH0203 | p35S2-GE1-modcrylAb-3'ocs<>p35S3-bar-3'nos | p35S-crylAb |
| PTSVH0207 | Pubil-ubi leader with intron-modcry1Ab-3'ocsop35S3-bar-3'nos | pUbi-crylAb |
| PTSVH0208 | Pgos2-cab22 leader-modcrylAb-3'ocsop35S3-bar-3'nos | pGos-cab-crylAb |
| PTSVH0209 | Pgos2-gos leader with intron-modcrylAb-3'ocsop35S3-bar-3'nos | pGos-gos-crylAb |
| PTSVH0212 | 3'nos-bar-p35S3><Tr2-modcrylAb-3'ocs | pTR2-crylAb |

| **Protoplast transformation** | | |
|---|---|---|
| PSVH0211 | Pubil-ubi leader with intron-crylAb53-3'ocsop35S3-bar-3'nos | pUbi-crylAb |
| PSVH0213 | Pgos2-gos leader with intron-modcry1Ab-3'ocs<>p35S3-bar-3'nos | pGos-gos-crylAb |

Regenerated plantlets were selected based on Liberty tolerance and/or measurement of PAT protein levels by ELISA (Clark et al, 1986).

### II. Evaluation of events

### a) general characterization

The *Agrobacterium* transformants were checked for presence of vector sequence at the left border of the T-DNA. Southern blot analyses were performed with leaf material of the primary transformants (T0).

### b) Expression of modified CrylAb protein

The events were analyzed in the greenhouse for CrylAb expression by detecting soluble modified CrylAb protein levels in different tissues by a Cry1Ab sandwich ELISA with a polycondensated IgG fraction of a polyclonal rabbit antiserum against CrylAb as first antibody and a monoclonal antibody against CrylAb as second antibody.

The expression levels of modified CrylAb protein in different tissues of early whorl plants transformed with the p35S-crylAb construct is provided in Table 1. Expression in the different progeny plants of one event obtained with p35S-crylAb event was found to be constitutive and above 0.5% on average.

**Table 1. Expression of CrylAb in different tissues of the early whorl plant**

| **Event** | **Plant no** | **Leaf blade** | **Root** |
|---|---|---|---|
| CP048-2602 (construct ptsvh0203) | 0042 | 0.73 | 1.4 |
| | 0032 | 1.12 | 3.18 |
| | 0033 | 1.14 | 2.39 |
| | 0034 | 1.12 | 2.65 |
| | 0035 | 0.94 | 3.51 |
| | 0039 | 0.88 | 2.48 |
| | 0040 | 0.68 | 1.7 |
| | 0036 | 0.27 | 0.81 |
| | 0037 | 0.13 | 0.66 |
| | 0038 | 0.15 | 1.15 |
| | 0041 | 0.13 | 0.49 |
| | 0043 | 0.08 | 0.78 |
| | 0044 | 0.13 | 0.98 |
| | 0045 | 0.08 | 0.55 |
| | 0031 | 0.11 | 0.57 |
| | Average (SD) | 0.51(0.44) | 1.55(1.03) |

The expression levels of CrylAb protein in different tissues in early V, R1 and R4-5 stage leaves, R4-5 stage kernels and in pollen for plants transformed with the p35S-crylAb, pGos-cab-crylAb, pGos-gos-cry1Ab and pTR2-cry1Ab constructs are provided in Table 2. Results are presented as averages of leaf and kernel samples taken from 5 plants and as averages of pollen samples taken from 3 plants (standard deviation in brackets). ICP expression for the one p35S-crylAb plant is above 0.1% total soluble protein. The p Gos-cab-crylAb plants showed around 0.01-0.06% CrylAb expression in leaves. The events obtained with the pGos-gos-cry1Ab constructs showed high expression (0.2-0.6% protein) in leaf tissue. Basal expression of protein in leaves of plants transformed with the modified crylAb DNA sequence under control of the wound-inducible TR2' promoter (pTR2'-crylAb) was low to undetectable.

**Table 2. Expression of Cryl Ab protein in different tissues of plants transformed with the CrylAb gene**

| | | Cry1Ab in % soluble protein / total protein | | | | |
|---|---|---|---|---|---|---|
| Event | Construct | 5 plants early V/leaf | 5 plants R1/leaf | 3plants pollen | 5 plants R4-5/leaf | 5 plants R4-5/kernel |
| CE048-2602 | p35S-cry1Ab | 0.186(0.066) | 0.54 (0.38) | 0 | 0.41(0.17) | 0.048(0.039) |
| CE104-0202 | pGos-cab-cry1Ab | 0.014(0.0055) | | | 0.036(0.015) | |
| CE1014-0402 | pGos-cab-cry1Ab | 0.032(0.016) | | | 0.041(0.011) | |
| CE122-0806 | pGos-cab-cry1Ab | 0.024(0.015) | 0.10 (0.013) | 0 | 0.058(0.036) | 0(0) |
| CE2162-0402 | pGos-gos-cry1Ab | 1.27(0.82) | | | | |
| CE2168-0202 | pGos-gos-cry1Ab | 0.467(0.23) | | | | |
| CE2168-1218 | pGos-gos-cry1Ab | 0.26(0.026) | | | | |
| CE21612-1402 | pGos-gos-cry1Ab | 0.158(0.067) | 0.256 (0.106) | 0 | 0.153(0.023) | 0.017(0.006) |
| CE21614-0604 | pGos-gos-cry1Ab | 0.682(0.50) | | | | |
| CE21614-0816 | pGos-gos-cry1Ab | 0.327(0.27) | 0.925 (0.198), 1 plant 0.06 | 0.036 (0.0115) | 0.44(0.078) | 0.0417(0.021) |
| CP21614-1606 | pGos-gos-cry1Ab | 0.38(0.153) | | | | |
| WI600-0218 | pTR2-cry1Ab | 0 | 0 | 0 | 0 | 0.002 |
| WI600-0802 | pTR2-cry1Ab | 0 | 0 | 0 | 0.0048(0.0016) | 0.01 |

In a greenhouse study, expression of modified CrylAb protein was determined in leaf samples of plants in V3 stage, and, where available, leaf and pollen of plants in R1 stage, and leaf, stalk and pollen of plants at harvest, for events obtained with the different constructs. Again, the plants with the pGos-gos-cry1Ab constructs showed high CrylAb expression in leaves (>0.2% total soluble protein) (irrespective of transformation method used). For these plants, high expression levels were also found in the stalk (0.8% total soluble protein or more on average). Events obtained from transformation with the pTR2'-crylAb constructs showed low (0.02% total soluble protein or less on average) or undetectable expression of CrylAb protein in all tissues tested.

For the events obtained with the pTR2'-cry1Ab construct, studies were performed in the greenhouse to determine the expression of the CrylAb protein upon mechanical damage. Leaf samples were taken before wounding, and 18h after mechanical damage and Cry1Ab protein levels were measured by ELISA (Table 3). Damaged leaf parts were excised and incubated for 18 hours in a petri dish on filter paper moistened with Murashige and Skoog (MS) medium (Plant Molecular Biology Labfax (1993) by R.D.D. Croy, jointly published by BIOS Scientific Publications Ltd (UK) and Blackwell Scientific Publications, UK) at room temperature and then Cry1Ab protein level was measured using a sandwich ELISA assay. Unwounded control leaf parts (of similar size as the wounded leaf parts) were excised from the plants and were immediately put on dry ice for protein analysis.

**Table 3: Expression of insecticidal protein in different plant parts before and after mechanical wounding**

| | | Cry1Ab in % soluble protein / total protein | | | | | | | | | |
|---|---|---|---|---|---|---|---|---|---|---|---|
| Event (construct) | | V4 stage | | Flowering | | | | | Harvest | | |
| | | Leaf | Leaf induced | Leaf | Leaf induced | root | Root induced | Pollen | Leaf | Stalk | Kernel |
| WI600-0218 | Mean | 0.000 | 0.020 | 0.000 | 0.020 | 0.023 | 0.036 | 0.000 | 0.000 | 0.000 | 0.000 |
| (pTR2'-Cry1Ab) | st.dev. | 0.000 | 0.008 | 0.000 | 0.007 | 0.008 | 0.011 | 0.000 | 0.000 | 0.000 | 0.000 |
| WI606-0406 | Mean | 0.005 | 0.046 | 0.000 | 0.007 | 0.012 | 0.008 | 0.000 | \ | 0.002 | 0.002 |
| (pTR2'-Cry1Ab) | st.dev. | 0.007 | 0.005 | 0.000 | 0.006 | 0.009 | 0.009 | 0.000 | | 0.004 | |
| WI604-1602 | Mean | 0.002 | 0.104 | 0.001 | 0.020 | 0.020 | 0.024 | 0.000 | 0.004 | 0.004 | 0.001 |
| (pTR2'-Cry1Ab) | st.dev. | 0.003 | 0.012 | 0.000 | 0.009 | 0.008 | 0.008 | 0.000 | 0.002 | 0.003 | 0.000 |
| WI606-0802 | Mean | 0.003 | 0.064 | 0.001 | 0.010 | 0.027 | 0.037 | 0.000 | 0.002 | 0.004 | 0.003 |
| (pTR2'-Cry1Ab) | st.dev. | | 0.004 | 0.000 | 0.009 | 0.012 | 0.020 | 0.000 | 0.001 | 0.003 | 0.001 |
| WI606-1206 | Mean | 0.001 | 0.081 | 0.000 | 0.022 | 0.032 | 0.024 | 0.000 | 0.004 | 0.002 | 0.001 |
| (pTR2'-Cry1Ab) | st.dev. | | 0.016 | 0.000 | 0.010 | 0.005 | 0.011 | 0.000 | 0.003 | 0.001 | 0.001 |
| CE048-2402 | Mean | 0.83 | 0.614 | 0.280 | 0.397 | 0.196 | 0.253 | 0.000 | 0.376 | 1.120 | 0.047 |
| (p35S-Cry1Ab) | st.dev. | 0.108 | 0.111 | 0.027 | | 0.133 | 0.083 | 0.000 | 0.098 | 0.175 | 0.014 |
| CE048-2602 | Mean | 0.636 | 0.527 | 0.007 | 0.006 | 0.087 | 0.045 | 0.000 | 0.106 | 0.040 | 0.015 |
| (p35S-Cry1Ab) | st.dev. | 0.16 | 0.046 | 0.002 | 0.002 | 0.044 | 0.007 | 0.000 | 0.011 | 0.013 | 0.004 |
| Control 1 | Mean | 0.000 | 0.000 | 0 | 0 | 0 | 0 | 0 | 0 | 0.001 | 0 |
| (untransformed) | st.dev. | 0.000 | 0.000 | 0 | 0 | 0 | 0 | 0 | 0 | 0.002 | 0 |
| Control 2 | Mean | 0.000 | 0.000 | 0 | 0 | 0 | 0 | 0 | 0 | 0 | 0 |
| (untransformed) | st.dev. | 0.000 | 0.000 | 0 | 0 | 0 | 0 | 0 | 0 | 0 | 0 |
| CE0104-0202 | Mean | 0.022 | 0.023 | 0.008 | 0.017 | 0.023 | 0.016 | 0.0001 | \ | \ | \ |
| (pGos/cab-Cry1 Ab) | st.dev. | 0.009 | 0.005 | 0.005 | 0.004 | 0.012 | 0.005 | 0.000 | | | |
| CE1014-0402 | Mean | 0.025 | 0.017 | 0.007 | 0.010 | 0.028 | 0.034 | 0.000 | 0.010 | 0.040 | 0.002 |
| (pGos/cab-Cryl Ab) | | 0.005 | 0.003 | 0.001 | 0.003 | 0.017 | 0.013 | 0.000 | 0.003 | 0.020 | 0.001 |

### c) Inducible Cry1Ab expression

Expression of the CrylAb protein was either absent or around the detection limit in leaves, stalk and kernels of the different pTR2' events tested. No expression above background levels (as found in untransformed control plants) was found in leaves and pollen in whorl and pollen shedding plant stage. Constitutive expression was seen in the roots. When leaves are mechanically damaged, expression of the modified Cry1Ab protein is induced and goes up to 0.02-0.1% in V4 stage leaves.

### d) ECB Efficacy

ECB efficacy trials were performed in the greenhouse and at two different locations in the field. At the same time, plants were evaluated for phytotoxicity effects of the constructs introduced. T able 4 shows the results of efficacy tested for ECB. In the greenhouse, ECB efficacy is determined by measuring the length of tunnels in cm per stalk for 10 plants and is expressed as the average length (sd in brackets) of tunnels per maximum number of tunnels per plant. In the field, ECB efficacy is expressed as the average of 3 values obtained for different groups of 10 plants.

**Table 4. ECB efficacy trials in the greenhouse and in different locations in the field**

| | **Greenhouse ECB efficacy** | **Field** 1 **ECB efficacy** | **Field 2 ECB Efficacy** |
|---|---|---|---|
| **p35S-cry1Ab** | | | |
| CE024-1301 | 1.2(1.47)/2 | 0.4(0.53)/4 | 0.05(0.07)/1 |

| **PGos-cab-cr1Ab** | | | |
|---|---|---|---|
| CE050-0802 | 0(0)/0 | 0(0)/0 | 0(0)/14 |
| CE052-0101 | 0.6(0.84)/2 | 0.37(0.43)/3 | 0.17(0.23)/2 |
| CE052-0205 | 1.6(2.4)/6 | 0.08(0.072)/1 | 0.05(0.07)/1 |
| CE053-0605 | 0.4(0.69)/2 | 0.113(0.1)/1 0.09(0.085)/1 | 0.047(0.08)/1 0(0)/0 |
| CE053-0702 | 0.6(0.96)/2 | 0.4(0.53)/5 | 0.055(0.097)1 |
| CE057-0201 | | 0.21(0.26)/1 | 0.2(0.35)/6 |
| CE060-0402 | 0.7(1.64)/5 | 0.91(0.44)4 | 0.4(0.692)/3 |
| CE061-0301 | 0.7(1.16)3 | 0.22(0.39)/4 | 0.0625(0.088)/1 |
| CE061-1102 | 2.7(1.76)/5 | 1 | |
| CE061-1402 | | 2.8(4.52)/8 | 0.22(0.47)/2 |
| CE061-1502 | 1.7(0.97)/(3) | 0.08(0.14)/1 | 0(0)/0 |
| CE062-0201 | | 0(0)/0 | 0(0)/0 |
| CE067-0401 | | 0(0)/0 | 0.205(0.113)1 |
| CE068-0903 | | 0.07(0.11)/2 | 0.04(0.0072)/1 |

| **pGos-gos-Cry1Ab** | | | |
|---|---|---|---|
| CE 180-0303 | 0(0)/0 | 0(0)/0 | 0(0)/0 |
| CE181-0201 | 0.1(0.32)/1 | 0.36(0.34)/2 | 0(0)/0 |
| CE182-0101 | | 0(0)/0 | 0.07(0.1)/0 |
| CE183-0501 | | 0(0)/0 | 00.056(0.08)/1 |
| CE183-0601 | | 1 | 0(0)/0 |
| Cue184-0101 | 0.7(1.06)/3 | 0.51(0.35)/2 | 0(0)/0 |
| CE184-0609 | | 0(0)/0 | 0(0)/0 |
| CE184-0801 | 0.11(0.33)/1 | 0(0)/0 | 0(0)/0 |
| CE186-0203 | | 0(0)/0 | 0.037(0.064)/1 |
| CE187-0302 | | 0.08(0.14)/2 | 0(0)/0 |
| CE187-0408 | 0.1(0.32)/1 | 0(0)/0 | 0.11(0.19)1 |
| CE187-0803 | | 0.096(0.17)/1 | 0.097(0.16) |
| CE193-1002 | | 0.15(0.17)/1 | 0.33(0.58)/4 |
| CE196-0201 | 0.4(1.26)/0 | 0(0)/0 | 0.144(0.14)1 |
| CE197-0102 | | 0(0)/0 | |
| CE198-0401 | 0.9(1.28)/3 | 0.09(0.08)/1 | 0(0)/0 |
| CE198-0802 | 0(0)/0 | 0.17(0.21)/3 | 0.03(0.057)/1 |
| CE198-1401 | 0(0)/0 | 0.047(0.0082)/1 | 0(0)/0 |
| CE198-1702 | 0.1(0.32)/1 | 0.55(0.74)/4 | 0(0)/0 |
| CE198-2101 | 0.1(0.32)/1 | 0(0)/0 | 0.085(0.12)1 |
| CE198-2501 | 0(0)/0 | 0(0)/0 | 0(0)/0 |

| **PUbi-crylAb** | | | |
|---|---|---|---|
| ACE054-00103 | 158.8(32.3)/200 | 19.0(1.4)30 | |
| ACE054-01502 | 166.2(31.2)/235 | 20.7(0.98)/27 | |
| ACE054-02803 | 114.5(92.1)/270 | 22.4(2.9)/42 | |
| B73 control | 150.6(40.3)/211 | 25.7(4.8)/39 | 27.6(7.4)/62 |

The p35S-crylAb event gave absolute ECB control, both in the greenhouse as in field-trials at different locations which correlated with the high-dose expression (as described above). Similarly, the pGos-gos-cry1Ab and PGos-cab-cry1Ab events displayed good ECB control at all locations tested. The pUbi-cry1Ab event did not show good ECB control.

No phytotoxicity was observed for any of the p35S-cry1Ab or pGos-cab-cry1Ab events. Selfed seed of pGos-gos-cry1Ab events showed segregation in the field of normal green plants and stunted, yellowish plants, from which the lower leaves are dying, suggestive of some impact on agronomic performance.

Fourteen events obtained with the pTR2'-cry1Ab construct were evaluated for ECB efficacy in the greenhouse and in field trials (Table 5). Four of the five single-copy events (indicated with an asterisk) gave total ECB2 control.

**Table 5: ECB Efficacy in the Greenhouse and in the Field for pTR2'-crylAb events**

| Event | ECB efficacy Greenhouse Average(sd)/max tunnels/pl | ECB efficacy Field average(sd)/max tunnels/pl | ECB efficacy Field Average(sd)/max tunnels/pl |
|---|---|---|---|
| WI602-0402 | 0.1(0.31)/1 | 0.24(0.41)/2 | 0.21(0.01)/2 |
| WI604-1602 | 0.2(0.42)/1 | 0.05(0.071)/1 | |
| WI606-0406* | 51.3(73.5)/195 | 8.41(1.8)/32 | 7.45(0.07)/30 |
| WI606-0802 | 3.3(2.6)/8 | 0(0)/0 | 0.05(0.07)/1 |
| WI606-1206 | 0.38(0.74)/2 | 0.17(0.29)/3 | 0.1(0.14)/1 |
| WI600-0218 | 2.0(1.87)/6 | | |
| WI600-1402* | 29.4(45.8)/142 | 6.55(2.47)/28 | |
| WI602-0202* | 168.5(26.9)/200 | 12.6(3.8)/31 | |
| WI604-0604* | 102.5(83)/251 | 3.7(2.0)/25 | |
| WI602-0802 | 0(0)/0 | 0.33(0.15)/3 | |
| WI602-0204 | 66(68.2)/160 | | |
| WI602-1002 | 102.4(63.4)/215 | | |
| WI606-0602 | 45.5(52.8)/160 | | |
| WI600-0802 | 0.9(1.44)/4 | 0.06(0.11 )/2 | 0(0)/0 |
| B73 control | 150.6(40.3)/211 | 25.7(4.8)/39 | 27.6(7.4)/62 |

No penalty on agronomic performance was observed for the plants after second selfing (ear to row) of the different TR2' events in any of the locations tested.

### e) Efficacy against Sesamia nonagrioides

Five mid whorl corn plants obtained with the pTR2'-cry1Ab construct were each infested with 2 egg masses. Damage was scored after 14 days and number of larvae were counted. Damage rating were averaged over the five plants. Non-transformed B73 plants were similarly infested as controls. Results are shown in Table 6.

**Table 6. Efficacy against Sesamia nonagrioides.**

| Event | Number of larvae per plant | Plant height in cm | Cm tunnels per plant |
|---|---|---|---|
| WI600-0802 | 0.6 (1.3) | 198 (8.4) | 0 |
| B73 Control | 197.2(14.0) | 84 (5.5) | 70 (9.4) |

### Example 3. Cry1Ab gene in cotton

### I. Development of CrylAb events in cotton

A construct was made for the expression of the modified cry1Ab gene in cotton. The pTSVH0203 construct contains the modified cry1Ab coding sequence (encoding part of the Cry1Ab protein described by Höfte et al.1986 having an insertion of an alanine codon (GCT) 3' of the ATG start codon) under control of the constitutive promoters 35S (Odell et al. 1985), linked to the leader sequence of the tapetum El gene from *Oryza sativa* (WO92/13956) with the 3' ocs terminator (fragment containing polyadenylation signals from the 3'untranslated region of the octopine synthase gene from the TL-DNA of pTiAch5, De Greve et al., 1983). The construct additionally comprises the *bar* coding sequence (the coding sequence of phosphinothricin acetyl transferase of Streptomyces hygroscopicus; Thompson et al., 1987) under control of the 35S promoter.

| | | |
|---|---|---|
| PTSVH0203 | P35S2-GE1-cry1Ab53-3'ocs<>p35S3-bar-3'nos | p35S-cry1Ab-ocs |

This construct was used for transformation of cotton. The obtained events were subjected to molecular analysis to confirm presence of the transgene.

### II. Analysis of events

### a) Expression of modified Cry1Ab protein

Expression of modified Cry1Ab protein was determined in leaf, terminal, square, flower and boll samples using ELISA (Table 7). Results represent percentage of Cry1Ab protein of total protein content as an average of samples taken from 9 plants. The time point at which samples were taken is represented as days after planting (in brackets).

**Table 7. Cry1Ab expression as measured by ELISA in different tissues**

| | ELISA , Cry1Ab expression in % , 9 plants | | | | | | | | |
|---|---|---|---|---|---|---|---|---|---|
| Events | Leaf (60) | Leaf (65-80) | Leaf (115-120) | Terminal leaf (60) | Terminal leaf (65-80) | Terminal leaf (115-120) | Squares (60) | Flower (65-80) | Bolls (115-120) |
| COCE040-04702A | 0.024 | 0.008 | 0.005 | 0.005 | 0.012 | 0.006 | 0.002 | 0.003 | 0 |
| COCE040-04702B | | 0.011 | | | | | | | |
| COCE040-3106-1143 | 0.017 | 0.022 | 0.012 | 0.015 | 0.011 | 0.007 | | 0.003 | |
| COCE040-3106-1144 | | 0.018 | | | | | | | |

### b) laboratory toxicity assay

CBW larvae were fed on squares, leaf and terminals obtained from leaves in the field. Weight of surviving larvae was measured for the different events (Table 8). Samples from two non-transgenic plants and from one plant comprising a herbicide resistance gene (LL25 event) were used as control.

**Table 8. Toxicity of modified Cry1Ab expressed in cotton to CBW larvae**

| | Weight in mg of surviving larvae | | |
|---|---|---|---|
| Event | Squares | Terminals | Leaf |
| 5 | 2.8 | 0 | 8.7 |
| 8 (- control LL25) | 22.3 | 1.7 | 15.5 |
| 11 | 0 | 0 | 18 |
| 13 | 4.3 | 1 | 20.6 |
| 16 | 6 | 0.3 | 23 |
| 21 (- control non-transgenic) | 16.5 | 8.7 | 26.3 |
| 23 (- control non-transgenic) | 8.2 | 38.5 | 29 |

### c) Efficacy of insect control in the field

In the field, plants were artificially infested with neonate CBW larvae. Damage to leaves, terminals, squares, white bloom and bolls was r ated and counts were made each 8 d ays i n August and September. A mean was made over the five observations dates. Statistical analysis indicated that significant differences were found in the mean damage severity of terminals, white blooms and bolls. Also, the mean number of damaged squares and damaged bolls was higher for the controls than for the Cry1Ab events; The mean number of larvae in squares was also significantly reduced compared to controls.

### Example 4. Cry1Ab gene in rice

A construct was made for the expression of the modified cry1Ab gene in rice. The construct contains the modified cry1Ab coding sequence (encoding part of the Cry1Ab5 protein described by Höfte et al.1986 having an insertion of an alanine codon (GCT) 3' of the ATG start codon) under control of the promoter with the 5' leader sequence of the GOS2 gene from rice, containing the second exon, the first intron and the first exon of the GOS transcript (de Pater et al., 1992). This construct was used for transformation of rice. The obtained events were subjected to molecular analysis to confirm presence of the Transgene.

Plants of 25 different events were tested for control of rice leaf folder, yellow stem borer, and pink stem borer by counting the number of surviving larvae. Damage to the plants was assessed as damage to the leaves (for leaf folder) or the number of deadhearts per number of tillers at the preflowering stage (yellow stem borer and p ink s tem borer) or the n umber of whiteheads per number of tillers at the flowering stage (yellow stem borer). For only 2 events plants were found on which some (5-7 out of 25) of the larvae survived. All plants of all other events showed 90-100% dead larvae. While the control plants were either completely dried up or showed many folded leaves when infested with rice leaf folder, none of the plants of the cry1Ab events showed any significant damage. Similarly, no deadhearts or whiteheads were detected for the plants of the cry1Ab e vents, infested with yellow stem borer or pink stem borer.

### References

Arvidson et al., 1989, Mol Microbiol 3:1533
Barton et al. 1987, Plant Physiol 85:1103-1109
Bates, 1995, Methods Cell Biol. 1995;50:363-73
Bogorad 2000, Trends Biotechn 18(6):257-263
Bradford, 1976, Anal. Biochem. 72: 248-254
Breitler et al. 2001, Mol Breeding 7: 259-274
Casas et al., 1995, Plant Breed Rev 13:235-264
Chen et al., 1994, Theor Appl Genet 88:187-192
Christensen et al. 1992, Plant Mol Biol 18(4):675-89
Christou, 1997, Plant Mol Biol 35(1-2):197-203
Clark et al., 1986, Methods Enzymol. 118:742-766.
Cordero et al. 1994, Plant J 6(2):141-50.
Cornelissen & Vandewiele, 1989, Nucleic Acids Research 17:19-23
Crickmore et al. 1995, 28th Annual Meeting of the Society for Invertebrate Pathology, Society for Invertebrate Pathology, Bethesda MD, p 14
D'Halluin et al., 1992, Plant Cell 4 : 1495-1505
Datta et al., 1999, Methods Mol Biol 111:335-347
De Block et al. 1989, Plant Physiol 914:694-701
De Greve et al., 1983, J Mol Applied Gen: 499-511
De maagd et al. 1999, Trends Plant Sci 4:9-13
de Pater et al., 1992, Plant J 2:837-844
Depicker et al., 1982, J Mol Appl Gen 1: 561-573
Duan et al., 1996, Nature Biotech 14:494-498
Dunwell, 1999, Methods Mol Biol 111: 375-82
Finer et al., 1999, Cur Top Micriobiol Immunol 240:59-80
Franck et al. 1980, Cell 21: 285-294
Grochulski et al., 1995, J Mol Biol 254:447
Guevara-Garcia et al., 1998, Plant J 4(3):495-505
Guthrie, 1989, Plant Breed Rev 6:209-243
Harpster et al. 1988, Mol Gen Genetics 212:182
Hiei et al., 1997, Plant Mol Biol 35:201-218
Hofte and Whiteley, 1989, Microbiol Rev 53(2):242-55
Höfte et al., 1986, Eur J Biochem 161:273-280
Hood et al., 1986. J. Bacterial. 168: 1291
Jansens et al., 1997, Crop Science 37(5):1616-1624
Johnson et al., 1989, PNAS 86:9871
Komari et al. 1998, Curr Opin Plant Biol 1(2):161-165
Langridge et al. 1989, Proc Natl Acad Sci USA 86:3219-3223
Newell, 2000, Mol Biotechnol 16(1):53-65
Odell et al., 1985, Nature 313: 810-812
Potrykus 1990, Cyba Found Symp 154:198-208
Poulsen, 1996, Plant Breeding 115:209-225
Sawahel et al. 1995, Biotechniques 19(1):106-110
Schnepf et al., 1998, Microbiol Mol Biol Rev 62:775-806
Thompson et al., 1987, EMBO J 6: 2519-2523
van der Salm et al., 1994, Plant Mol Biol 26:51
Vasil et al. 1999, Methods Mol Biol 111:349-358
Velten et al. 1984, EMBO J. 12:2733-2730
Wilbur and Lipmann, 1983, Proc Natl Acad Sci U.S.A. 80:726 Witowsky & Siegfried, 1997, PBI Bulletin 14-15

## Claims

1. A DNA sequence comprising a coding region encoding a modified Cry1Ab protein comprising the nucleotide sequence of SEQ ID NO: 2.

2. A chimeric gene comprising the DNA sequence of claim 1 under control of a plant-expressible promoter.

3. A recombinant vector comprising the chimeric gene of claim 2.

4. A transgenic plant cell comprising the chimeric gene of claim 2.

5. A transgenic plant comprising the plant cell of claim 4.

6. The transgenic plant of claim 5, which is selected from corn, cotton, or rice.

7. A seed of the plant of claim 6, which comprises the DNA sequence of SEQ ID NO:2.

8. A method for engineering insect resistance in a plant, so as to protect said plant from lepidopteran insects pests, comprising introducing by transforming into the genome of said plant the chimeric gene of claim 2.

9. The method of claim 8, wherein said insect pests are selected from the group of European Corn Borer, *Sesamia nonagrioides,* cotton bollworm, tobacco budworm, yellow stem borer, pink stem borer, and the rice leaf folder.

10. Use of the chimeric gene of claim 2 to obtain insect-resistant plants.

## Patentansprüche

1. DNA-Sequenz, umfassend eine Codierregion die für ein modifiziertes Cry1Ab-Protein codiert, und umfassend die Nukleotidsequenz gemäß SEQ ID Nr.: 2.

2. Chimäres Gen, umfassend die DNA-Sequenz nach Anspruch 1 unter der Kontrolle eines in Pflanzen exprimierbaren Promoters.

3. Rekombinanter Vektor, umfassend das chimäre Gen nach Anspruch 2.

4. Transgene Pflanzenzelle, umfassend das chimäre Gen nach Anspruch 2.

5. Transgene Pflanze, umfassend die Pflanzenzelle nach Anspruch 4.

6. Transgene Pflanze nach Anspruch 5, ausgewählt aus der Reihe Mais, Baumwolle bzw. Reis.

7. Samen der Pflanze nach Anspruch 6, der die DNA-Sequenz nach SEQ ID Nr.: 2 umfaßt.

8. Verfahren zur Erzeugung von Insektenresistenz in einer Pflanze, um die Pflanze gegen Lepidopteren-Schadinsekten zu schützen, umfassend das Einführen des chimären Gens nach Anspruch 2 in das Genom der Pflanze durch Transformation.

9. Verfahren nach Anspruch 8, wobei die Schadinsekten aus der Gruppe Maiszünsler, *Sesamia nonagrioides,* Baumwollkapselwurm, Tabakknospenwurm, Gelber Stengelbohrer, Rosa Stengelbohrer und Reisblattwickler stammen.

10. Verwendung des chimären Gens nach Anspruch 2 zur Gewinnung von insektenresistenten Pflanzen.

## Revendications

1. Séquence d'ADN comprenant une région codante codant pour une protéine Cry1Ab modifiée comprenant la séquence nucléotidique de SEQ ID NO : 2.

2. Gène chimère comprenant la séquence d'ADN selon la revendication 1 sous le contrôle d'un promoteur exprimable chez les plantes.

3. Vecteur recombinant comprenant le gène chimère selon la revendication 2.

4. Cellule végétale transgénique comprenant le gène chimère selon la revendication 2.

5. Plante transgénique comprenant la cellule végétale selon la revendication 4.

6. Plante transgénique selon la revendication 5, qui est choisie parmi le maïs, le coton et le riz.

7. Graine de la plante selon la revendication 6, qui comprend la séquence d'ADN de SEQ ID NO : 2.

8. Méthode de création par génie génétique d'une résistance aux insectes chez une plante, de manière à protéger ladite plante des insectes nuisibles lépidoptères, comprenant l'introduction par transformation dans le génome de ladite plante du gène chimère selon la revendication 2.

9. Méthode selon la revendication 8, selon laquelle lesdits insectes nuisibles sont choisis dans le groupe constitué de la pyrale du maïs, de *Sesamia nonagrioides,* du ver de la capsule du cotonnier, de la noctuelle verdoyante, de la pyrale jaune du riz, de la noctuelle du riz et de l'enrouleuse des feuilles.

10. Utilisation du gène chimère selon la revendication 2, en vue d'obtenir des plantes résistantes aux insectes.


### SEQUENCE LISTING

<110> Bayer Bioscience N.V.
   <120> Insect resistant plants and methods for making same
   <130> MOD1AB WO1
<150> US 10/137682
   <151> 2002-05-03
<160> 2
<170> PatentIn version 3.2
<210> 1
   <211> 617
   <212> PRT
   <213> Artificial: modified protein from Bacillus thuringiensis
<400> 1
   Met Ala Asp Asn Asn Pro Asn Ile Asn Glu Cys Ile Pro Tyr Asn Cys
   1               5                   10                  15
   Leu Ser Asn Pro Glu Val Glu Val Leu Gly Gly Glu Arg Ile Glu Thr
               20                  25                  30
   Gly Tyr Thr Pro Ile Asp Ile Ser Leu Ser Leu Thr Gln Phe Leu Leu
           35                  40                  45
   Ser Glu Phe Val Pro Gly Ala Gly Phe Val Leu Gly Leu Val Asp Ile
       50                  55                  60
   Ile Trp Gly Ile Phe Gly Pro Ser Gln Trp Asp Ala Phe Leu Val Gln
   65                  70                  75                  80
   Ile Glu Gln Leu Ile Asn Gln Arg Ile Glu Glu Phe Ala Arg Asn Gln
                   85                  90                  95
   Ala Ile Ser Arg Leu Glu Gly Leu Ser Asn Leu Tyr Gln Ile Tyr Ala
               100                 105                 110
   Glu Ser Phe Arg Glu Trp Glu Ala Asp Pro Thr Asn Pro Ala Leu Arg
           115                 120                 125
   Glu Glu Met Arg Ile Gln Phe Asn Asp Met Asn Ser Ala Leu Thr Thr
       130                 135                 140
   Ala Ile Pro Leu Phe Ala Val Gln Asn Tyr Gln Val Pro Leu Leu Ser
   145                 150                 155                 160
   Val Tyr Val Gln Ala Ala Asn Leu His Leu Ser Val Leu Arg Asp Val
                   165                 170                 175
   Ser Val Phe Gly Gln Arg Trp Gly Phe Asp Ala Ala Thr Ile Asn Ser
               180                 185                 190
   Arg Tyr Asn Asp Leu Thr Arg Leu Ile Gly Asn Tyr Thr Asp His Ala
           195                 200                 205
   Val Arg Trp Tyr Asn Thr Gly Leu Glu Arg Val Trp Gly Pro Asp Ser
       210                 215                 220
   Arg Asp Trp Ile Arg Tyr Asn Gln Phe Arg Arg Glu Leu Thr Leu Thr
   225                 230                 235                 240
   Val Leu Asp Ile Val Ser Leu Phe Pro Asn Tyr Asp Ser Arg Thr Tyr
                   245                 250                 255
   Pro Ile Arg Thr Val Ser Gln Leu Thr Arg Glu Ile Tyr Thr Asn Pro
               260                 265                 270
   Val Leu Glu Asn Phe Asp Gly Ser Phe Arg Gly Ser Ala Gln Gly Ile
           275                 280                 285
   Glu Gly Ser Ile Arg Ser Pro His Leu Met Asp Ile Leu Asn Ser Ile
       290                 295                 300
   Thr Ile Tyr Thr Asp Ala His Arg Gly Glu Tyr Tyr Trp Ser Gly His
   305                 310                 315                 320
   Gln Ile Met Ala Ser Pro Val Gly Phe Ser Gly Pro Glu Phe Thr Phe
                   325                 330                 335
   Pro Leu Tyr Gly Thr Met Gly Asn Ala Ala Pro Gln Gln Arg Ile Val
               340                 345                 350
   Ala Gln Leu Gly Gln Gly Val Tyr Arg Thr Leu Ser Ser Thr Leu Tyr
           355                 360                 365
   Arg Arg Pro Phe Asn Ile Gly Ile Asn Asn Gln Gln Leu Ser Val Leu
       370                 375                 380
   Asp Gly Thr Glu Phe Ala Tyr Gly Thr Ser Ser Asn Leu Pro Ser Ala
   385                 390                 395                 400
   Val Tyr Arg Lys Ser Gly Thr Val Asp Ser Leu Asp Glu Ile Pro Pro
                   405                 410                 415
   Gln Asn Asn Asn Val Pro Pro Arg Gln Gly Phe Ser His Arg Leu Ser
               420                 425                 430
   His Val Ser Met Phe Arg Ser Gly Phe Ser Asn Ser Ser Val Ser Ile
           435                 440                 445
   Ile Arg Ala Pro Met Phe Ser Trp Ile His Arg Ser Ala Glu Phe Asn
       450                 455                 460
   Asn Ile Ile Pro Ser Ser Gln Ile Thr Gln Ile Pro Leu Thr Lys Ser
   465 470 475 480
   Thr Asn Leu Gly Ser Gly Thr Ser Val Val Lys Gly Pro Gly Phe Thr
   485 490 495
   Gly Gly Asp Ile Leu Arg Arg Thr Ser Pro Gly Gln Ile Ser Thr Leu
   500 505 510
   Arg Val Asn Ile Thr Ala Pro Leu Ser Gln Arg Tyr Arg Val Arg Ile
   515 520 525
   Arg Tyr Ala Ser Thr Thr Asn Leu Gln Phe His Thr Ser Ile Asp Gly
   530 535 540
   Arg Pro Ile Asn Gln Gly Asn Phe Ser Ala Thr Met Ser Ser Gly Ser
   545 550 555 560
   Asn Leu Gln Ser Gly Ser Phe Arg Thr Val Gly Phe Thr Thr Pro Phe
   565 570 575
   Asn Phe Ser Asn Gly Ser Ser Val Phe Thr Leu Ser Ala His Val Phe
   580 585 590
   Asn Ser Gly Asn Glu Val Tyr Ile Asp Arg Ile Glu Phe Val Pro Ala
   595 600 605
   Glu Val Thr Phe Glu Ala Glu Tyr Asp
   610 615
<210> 2
   <211> 1854
   <212> DNA
   <213> Artificial: sequence encoding modified Bacillus thuringiensis protein
<400> 2
   atggctgaca acaaccccaa catcaacgag tgcatcccct acaactgcct gagcaaccca 60
   gaggtggagg tgctgggtgg tgagaggatc gagaccggtt acacccccat cgacatcagc 120
   ctgagcctga cccagttcct gctgagcgag ttcgtgcctg gtgctggctt cgtgctggga 180
   ctagtggaca tcatctgggg catcttcggt cccagccagt gggatgcctt cctggtgcag 240
   atcgaacagt taattaacca aagaatagaa gaattcgcta ggaaccaagc catctctaga 300
   ctggagggcc tgagcaacct gtaccagatc tacgccgaga gcttccgcga gtgggaggct 360
   gaccccacca acccagccct gcgcgaggag atgcgcatcc agttcaacga catgaactct 420
   gccctgacca ccgccatccc actcttcgct gtccagaact accaggtccc tctcctgtct 480
   gtctatgtgc aagctgccaa cctccatctc agcgtccttc gcgacgtgag cgtctttggg 540
   cagaggtggg ggttcgacgc tgccaccatc aacagccgct acaacgacct gacgcgtctg 600
   atcggcaact acaccgacca cgcagtgaga tggtacaaca ctgggcttga gagggtctgg 660
   ggtcccgaca gccgcgactg gatcaggtac aaccagttca ggcgtgaact cactctcacc 720
   gtcttggata tcgtcagtct cttccccaac tacgacagca ggacctaccc tatccggact 780
   gtgagccagc tgacccgcga gatctacacc aaccccgtgc tggagaactt cgacggcagc 840
   ttcaggggct ctgcccaggg catcgagggc agcatccgca gcccccacct gatggacatc 900
   ctgaacagca tcaccatcta cactgacgcc cacaggggtg agtactactg gtctggccac 960
   cagatcatgg cttctcccgt gggcttcagc ggtcccgagt tcaccttccc cctgtacggc 1020
   acaatgggca acgctgcccc acagcagagg atcgtggccc agctgggcca gggcgtgtac 1080
   cgcaccctga gcagcaccct gtacaggagg cccttcaaca tcggcatcaa caaccagcag 1140
   ctgagcgtgc tggatggcac cgagttcgcc tacggcacca gcagcaacct gcccagcgcc 1200
   gtataccgca agagcggcac tgtggacagc ctggacgaga tcccacccca gaacaacaac 1260
   gtgcccccta ggcaggggtt ctctcatcgc ctctcacacg tgagcatgtt ccgcagcggc 1320
   ttcagcaaca gcagcgtgag catcatcagg gctcccatgt tcagctggat ccaccgcagc 1380
   gctgagttca acaacatcat tccaagtagc cagatcactc agatcccact caccaagagc 1440
   accaacctgg gctccgggac tagcgttgtc aagggaccag ggttcactgg aggcgacatc 1500
   ctgaggagga ccagcccagg ccagatcagc accttaaggg tgaacatcac cgctcccctc 1560
   agccaacgct acagggtcag gatcaggtac gcttccacca ccaacctgca gttccacacc 1620
   agcatcgacg gcaggcccat caaccagggc aacttcagcg ccaccatgag cagcggcagc 1680
   aacctgcaga gcggaagctt ccgcactgtg ggcttcacta ccccattcaa cttctccaac 1740
   ggcagcagcg tgttcaccct gtctgcccac gtgttcaaca gcggcaacga ggtgtacatc 1800
   gacaggatcg agtttgtccc agctgaggtg accttcgaag ctgagtacga ctga 1854